# EUROPEAN PATENT APPLICATION

(11) **EP 4 792 765 A1**
(43) Date of publication of application: **19.08.2026**
(21) Application number: 24891550.6
(22) Date of filing: 27.03.2024
(51) Int. Cl.: A61B 34/10, A61B 3/107, A61B 3/00, A61B 34/00, A61F 2/14, A61F 2/16, A61F 9/007, G16H 50/20

(54) **METHOD, DEVICE, AND COMPUTER PROGRAM FOR PROVIDING GUIDE FOR MULTIFOCAL INTRAOCULAR LENS IMPLANTATION ON BASIS OF ANALYSIS OF PATIENT'S CORNEAL CONDITION**

(30) Priority: 13.11.2023 KR 20230156571; 25.03.2024 KR 20240040133
(71) Applicant: Chois Young Vision Lab Inc., Seoul 06011 (KR)
(72) Inventor: YOON, Sooyoung, Seoul 06006 (KR); CHOI, Sungho, Seoul 06006 (KR); CHOI, Yoonseong, Seoul 06006 (KR); CHOI, Yoolim, Seoul 06006 (KR)
(74) Representative: Kim Kang, Jae Hee
(86) International application number: PCT/KR2024/003864
(87) International publication number: WO 2025/105601

(57) **Abstract**

Provided are a method, a device, and a computer program for providing a guide for multifocal intraocular lens implantation on the basis of an analysis of a patient's corneal state. The method for providing a guide for multifocal intraocular lens implantation on the basis of an analysis of a patient's corneal state, which is performed by a computing device, comprises the steps of: obtaining ocular data generated by scanning a patient's ocular area; analyzing the obtained ocular data to determine the patient's corneal state; and providing a guide for multifocal intraocular lens implantation to be performed on the patient on the basis of the determined corneal state of the patient.

## Description

### [Technical Field]

The present invention relates to a method, a device, and a computer program for providing guidance on multifocal intraocular lens implantation on the basis of analysis of a patient's corneal state.

### [Background Art]

In the field of cataract surgery, recent advancements in technology aimed not only at restoring patients' vision but also at improving the quality of their vision are attracting attention. One such technique is cataract surgery employing multifocal intraocular lenses. Multifocal intraocular lenses are designed to enable patients to see clearly at near, intermediate, and far distances, significantly expanding the range of vision compared to conventional monofocal intraocular lenses. For this reason, many patients have been able to enjoy a better quality of life.

However, for the implantation of these multifocal intraocular lenses to yield successful results, several prerequisites should be met. One important prerequisite is that the patient's eye should retain its normal shape and function excluding the cataract. Among these factors, the health and function of the cornea play a decisive role in the success of the surgery. The cornea is located at the forefront of the eye and plays an important role in refracting light to focus the light on the retina. When the shape and refractive power of the cornea are not suitable, it is difficult to achieve an expected improvement in vision even with implantation of a multifocal intraocular lens.

Currently, corneal topography systems are widely used as diagnostic equipment for assessing the optical function of the cornea. This equipment is necessary for precisely measuring the shape of the cornea and diagnosing corneal refractive errors. However, current corneal topography analysis methods have some limitations. First, there are no clear and specific criteria for determining which factors in the analysis results actually affect vision. For this reason, it is difficult to accurately assess the condition of the cornea and determine a surgical plan. Second, current methods primarily focus on data within a 6 mm diameter at the center of the cornea, and there is a lack of methods of comprehensively assessing the overall optical function of not only the central cornea but also the peripheral cornea at a time. This limits the comprehensive understanding of the overall condition of the cornea and may be particularly problematic when precise vision correction such as multifocal intraocular lens implantation is required.

The background art described above has been gained or acquired by the inventor in the course of deriving the subject matter of the present disclosure and is not necessarily considered prior art disclosed to the general public prior to the filing of this application.

### [Disclosure]

### [Technical Problem]

Objects to be achieved by the present invention are to address the conventional issues described above, and the present invention is directed to providing a method, a device, and a computer program for providing guidance on multifocal intraocular lens implantation on the basis of analysis of a patient's corneal state that determine the patient's corneal state accurately and precisely, provide guidance related to multifocal intraocular lens implantation on the basis of the results, and thereby increase the success rate of cataract surgery employing a multifocal intraocular lens and significantly contribute to improving the patient's vision and quality of life.

Objects to be achieved by the present invention are not limited to that described above, and other objects that have not been described will be clearly understood by those of ordinary skill in the art from the following description.

### [Technical Solution]

To achieve the above-described purpose, a method of providing guidance on multifocal intraocular lens implantation on the basis of analysis of a patient's corneal state according to an embodiment of the present invention, which is performed by a computing device, includes acquiring ocular data generated by scanning a patient's ocular area, determining the patient's corneal state by analyzing the obtained ocular data, and providing guidance on multifocal intraocular lens implantation to be performed on the patient on the basis of the determined corneal state of the patient.

According to various embodiments, the determining of the patient's corneal state may include calculating a metric related to irregularity of a corneal surface by analyzing the acquired ocular data and determining the patient's corneal state using the calculated metric.

According to various embodiments, the calculating of the metric may include generating a plurality of sections by dividing the patient's corneal area at predetermined intervals on the basis of a corneal apex or a pupil center and calculating a plurality of high-order aberrations (HOAs) for each of the plurality of generated sections as the metric related to the irregularity of the corneal surface.

According to various embodiments, the calculating of the metric may include generating a plurality of sections by dividing the patient's corneal area at predetermined intervals on the basis of a corneal apex or a pupil center, calculating a plurality of HOAs for each of the plurality of generated sections, and calculating an HOA change between adjacent sections as the metric related to the irregularity of the corneal surface.

According to various embodiments, the determining the patient's corneal state using the calculated metric may include calculating a plurality of reference metrics using a plurality of pieces of ocular data generated by scanning eyes of healthy people with no history of ophthalmic surgery and setting a normal range using the plurality of calculated reference metrics, and determining the patient's corneal state depending on whether the calculated metric is within the set normal range.

According to various embodiments, the determining of the patient's corneal state depending on whether the calculated metric is within the set normal range may include determining the patient's corneal state as a normal state when the calculated metric is within the set normal range and determining the patient's corneal state as an abnormal state when the calculated metric deviates from the set normal range. Here, the patient's corneal state may be determined as a caution state when the calculated metric is a threshold or less, and may be determined as a danger state when the calculated metric exceeds the threshold.

According to various embodiments, the determining the patient's corneal state using the calculated metric may include calculating a plurality of reference metrics using a plurality of pieces of ocular data generated by scanning eyes of healthy people with no history of ophthalmic surgery and generating a healthy-group normal distribution using the plurality of calculated reference metrics, and standardizing the calculated metric and determining the patient's corneal state on the basis of a location of the standardized metric in the generated healthy-group normal distribution.

According to various embodiments, the providing of the guidance may include determining prognosis of the multifocal intraocular lens implantation on the basis of the determined corneal state and providing guidance for information on the determined prognosis.

According to various embodiments, the providing of the guidance may include, when the patient's corneal state is determined as an abnormal state on the basis of the determined corneal state, providing guidance recommending the patient refractive surgery before the multifocal intraocular lens implantation.

According to various embodiments, the providing of the guidance may include providing guidance on use of an intraocular lens for the multifocal intraocular lens implantation on the basis of the determined corneal state. Here, guidance on cautious use of a diffractive intraocular lens may be provided when the determined corneal state is a caution state, and guidance on cautious use of any intraocular lens may be provided when the determined corneal state is a warning state.

According to various embodiments, the providing of the guidance may include providing a user interface (UI) for visualizing comparison results between the determined corneal state and corneal states of healthy people with no history of ophthalmic surgery and outputting the visualized comparison results.

To achieve the above-described purpose, a computing device for performing a method of providing guidance on multifocal intraocular lens implantation on the basis of analysis of a patient's corneal state according to another embodiment of the present invention includes a processor, a network interface, a memory, and a computer program loaded into the memory and executed by the processor. The computer program may include an instruction to acquire ocular data generated by scanning a patient's ocular area, an instruction to determine the patient's corneal state by analyzing the obtained ocular data, and an instruction to provide guidance on multifocal intraocular lens implantation to be performed on the patient on the basis of the determined corneal state of the patient.

To achieve the above-described purpose, a computer program according to still another embodiment of the present invention is stored on a computer-readable recording medium to perform, in combination with a computing device, a method of providing guidance on multifocal intraocular lens implantation on the basis of analysis of a patient's corneal state, the method including acquiring ocular data generated by scanning a patient's ocular area, determining the patient's corneal state by analyzing the obtained ocular data, and providing guidance on multifocal intraocular lens implantation to be performed on the patient on the basis of the determined corneal state of the patient.

Other details of the present invention are included in the detailed description and drawings.

### [Advantageous Effects]

According to various embodiments of the present invention, a patient's corneal state is accurately and precisely determined, and guidance related to multifocal intraocular lens implantation is provided on the basis of the result, thereby increasing the success rate of cataract surgery employing a multifocal intraocular lens and significantly contributing to improving the patient's vision and quality of life.

Effects of the present invention are not limited to that described above, and other effects that have not been described will be clearly understood by those of ordinary skill in the art from the following description.

### [Description of Drawings]

The following drawings attached to this specification illustrate exemplary embodiments of the present invention and serve to facilitate understanding the technical concept of the present invention in conjunction with the detailed description. of the invention. Therefore, the present invention should not be construed as being limited solely to the matters described in the drawings.
FIG. 1 is a diagram showing a system for providing guidance on multifocal intraocular lens implantation on the basis of analysis of a patient's corneal state according to an embodiment of the present invention.
FIG. 2 is a hardware configuration diagram of a computing device for performing a method of providing guidance on multifocal intraocular lens implantation on the basis of analysis of a patient's corneal state according to another embodiment of the present invention.
FIG. 3 is a flowchart illustrating a method of providing guidance on multifocal intraocular lens implantation on the basis of analysis of a patient's corneal state according to still another embodiment of the present invention.
FIG. 4 is a flowchart illustrating a method of calculating a metric for determining a patient's corneal state according to various embodiments.
FIG. 5 schematically shows a user interface (UI) for selecting at least one of a plurality of different types of metrics according to various embodiments
FIGS. 6 to 10 are diagrams schematically showing UIs for visually outputting comparison results between a patient's corneal state and healthy people's corneal states according to various embodiments.

### [Modes of the Invention]

The advantages and features of the present invention and a method of achieving them will become apparent from embodiments which will be described in detail below with reference to the accompanying drawings. However, the present invention is not limited to the embodiments disclosed below and may be implemented in various different forms. The embodiments are only provided to make the disclosure of the present invention complete and fully convey the scope of the present invention to those skilled in the technical field to which the present invention pertains. The present invention is only defined by the scope of the claims.

Terminology used in this specification is only for the purpose of describing embodiments and is not intended to limit the present invention. In this specification, singular forms include plural forms as well unless the context particularly indicates otherwise. The terms "comprises" and/or "comprising" used in this specification do not preclude the presence or addition of one or more components other than stated components.

Throughout the specification, like reference numerals refer to like components, and the term "and/or" includes any of stated components or any combination thereof. Although the terms "first," "second," etc., are used to describe various components, these components are not limited by the terms. These terms are used for the sole purpose of distinguishing one component from others. Therefore, a first component mentioned below may be a second component within the technical scope of the present invention.

As used herein, the term "unit" or "module" refers to a software component or a hardware component such as a field programmable gate array (FPGA) or an application-specific integrated circuit (ASIC), and a "unit" or "module" performs certain roles. However, a "unit" or "module" is not limited to software or hardware. A "unit" or "module" may be configured to reside in an addressable storage medium or reproduce one or more processors. Accordingly, as an example, a "unit" or "module" includes components, such as software components, object-oriented software components, class components, and task components, processes, functions, attributes, procedures, subroutines, segments of program code, drivers, firmware, microcode, circuitry, data, databases, data structures, tables, arrays, and variables. Components and functions provided in "units" or "modules" may be combined into a smaller number of components, and "units" or "modules" may be subdivided into additional components and "units" or "modules."

Spatially relative terms "below," "beneath," "lower," "above," "upper," etc., may be used to easily describe the relationship between one component and others shown in the drawings. Spatially relative terms should be understood as terms that include different directions of components during use or operation in addition to directions shown in the drawings. For example, when a component shown in the drawings is turned over, the component described as "below" or "beneath" another component may be placed "above" the other component. Accordingly, the exemplary term "below" may include both directions, below and above. Components may also be oriented in other directions, and spatially relative terms may be interpreted according to orientation.

In naming the same kind of plurality of targets, the terms "first," "second," etc., used herein are only used for the purpose of distinguishing one target from others and do not limit the order, importance, etc., of targets unless described otherwise.

As used herein, each expression such as "A, B, and C," "A, B, or C," "A, B, and/or C," "at least one of A, B, or C," at least one of A, B, and/or C," "at least selected one from A, B, and C," "at least selected one of A, B, or C," "at least selected one of A, B, and/or C," etc., may include any one of items listed with the expression or all possible combinations thereof. For example, the expression "at least selected one from A and B" may be (1) A, (2) at least one of A, (3) B, (4) at least one of B, (5) at least one of A and at least one of B, (6) B and at least one of A, (7) A and at least one of B, or (8) both A and B.

As used herein, the expression "based on" is used to describe one or more factors that influence the action or process of making a decision or judgment described in the phrase or sentence including the expression, and this expression does not preclude additional factors that influence the action or process of making a decision or judgment.

In this specification, when a component (e.g., a first component) is referred to as being "connected" or "coupled" to another component (e.g., a second component), the component may be directly connected or coupled to the other component, or the component may be connected or coupled to the other component by means of a new component (e.g., a third component).

As used herein, the expression "configured to" may have meanings of "set to," "capable of," "changed to," "made to," "able to," etc., depending on the context. The expression is not limited to the meaning "specially designed as hardware." For example, a processor configured to perform a specific operation may be a generic-purpose processor that may perform the specific operation by executing software.

Unless defined otherwise, all terms used herein (including technical or scientific terms) have the same meanings as those generally understood by those skilled in the technical field to which the present invention pertains. In addition, terms defined in commonly used dictionaries are not interpreted ideally or excessively unless clearly and particularly defined herein.

In this specification, a computer is any type of hardware device including at least one processor and may be understood as collectively including a software element operating in the corresponding hardware device depending on embodiments. For example, a computer may be understood as, but is not limited to, a meaning including all of a smartphone, a tablet personal computer (PC), a desktop computer, a notebook computer, and a user client and an application running on each device.

Hereinafter, embodiments of the present invention will be described in detail with reference to the accompanying drawings.

Although steps described in this specification are described as being performed by a computer, an entity performing each step is not limited thereto, and at least some steps may be performed on different devices depending on embodiments.

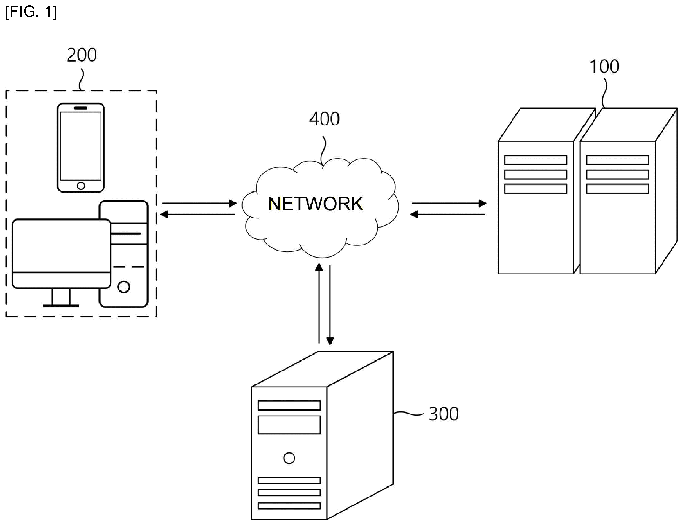

FIG. 1 is a diagram showing a system for providing guidance on multifocal intraocular lens implantation on the basis of analysis of a patient's corneal state according to an embodiment of the present invention.

Referring to FIG. 1, a system for providing guidance on multifocal intraocular lens implantation on the basis of analysis of a patient's corneal state according to the embodiment of the present invention may include a computing device 100, a user terminal 200, an external server 300, and a network 400.

The system for providing guidance on multifocal intraocular lens implantation on the basis of analysis of a patient's corneal state shown in FIG. 1 is in accordance with the embodiment. The components are not limited to those of the embodiment shown in FIG. 1, and as necessary, the components may be added, changed or removed.

According to the embodiment, the computing device 100 may provide a multifocal intraocular lens implantation guidance service on a request acquired from a user.

Here, the user may be a medical worker who performs multifocal intraocular lens implantation. However, the user is not limited thereto and may be a patient on whom multifocal intraocular lens implantation is performed.

Also, the multifocal intraocular lens implantation guidance service may be a service that analyzes a patient's corneal state and provides a variety of solutions and guidance related to the multifocal intraocular lens implantation on the basis of the analysis for the purpose of increasing the success rate of the multifocal intraocular lens implantation to be performed on the patient.

As an example, the computing device 100 may determine prognosis of the multifocal intraocular lens implantation that will be performed on the patient on the basis of a result of determining the patient's corneal state and provide the multifocal intraocular lens implantation guidance service for providing the determined prognosis.

As another example, the computing device 100 may recommend a suitable intraocular lens for the patient on the basis of the result of determining the patient's corneal state or provide the multifocal intraocular lens implantation guidance service for providing guidance on intraocular lenses that require caution for use.

As still another example, the computing device 100 may provide the multifocal intraocular lens implantation guidance service for visualizing comparison results between the patient's corneal state and healthy people's corneal states and providing the visualized comparison results.

In addition, the multifocal intraocular lens implantation guidance service may be a service that provides various kinds of information such as diagnostic results for eyes requiring treatment for irregular astigmatism or comparison results between the patient's pre- and posttreatment outcomes.

According to various embodiments, the computing device 100 may be connected to the user terminal 200 via the network 400 and provide the multifocal intraocular lens implantation guidance service to the user terminal 200 on a service provision request acquired from the user terminal 200.

The multifocal intraocular lens implantation guidance service provided by the computing device 100 may be, but is not limited to, implemented in a form accessible via a web-based interface or implemented as application software in the form of a mobile and/or desktop application.

The user terminal 200 may be any form of entity in a system with a mechanism for communication with the computing device 100. For example, the user terminal 200 may encompass a PC, a notebook computer, a mobile terminal, a smartphone, a tablet PC, a wearable device, etc., and encompass any kind of terminal that may access a wired/wireless network. Also, the user terminal 200 may encompass any computing device implemented by at least one of an agent, an application programming interface (API), and a plug-in. In addition, the user terminal 200 may include an application source and/or a client application.

The network 400 may be a connective architecture that enables information exchange between nodes such as a plurality of terminals and servers. For example, the network 400 may encompass a local area network (LAN), a wide area network (WAN), the Internet (the world wide web (WWW)), a wired or wireless data communication network, a telephone network, a wired or wireless television network, a controller area network (CAN), an Ethernet network, and the like.

The wireless data communication network may encompass, but is not limited to, a 3^{rd} Generation (3G) network, a 4^{th} Generation (4G) network, a 5^{th} Generation (5G) network, a 3^{rd} Generation Partnership Project (3GPP) network, a 5^{th} Generation Partnership Project (5GPP) network, a Long Term Evolution (LTE) network, a World Interoperability for Microwave Access (WiMAX) network, a Wi-Fi network, the Internet, a LAN, a wireless LAN, a WAN, a personal area network (PAN), a radio frequency (RF) network, a Bluetooth network, a near-field communication (NFC) network, a satellite broadcast network, an analog broadcast network, a digital multimedia broadcasting (DMB) network, and the like.

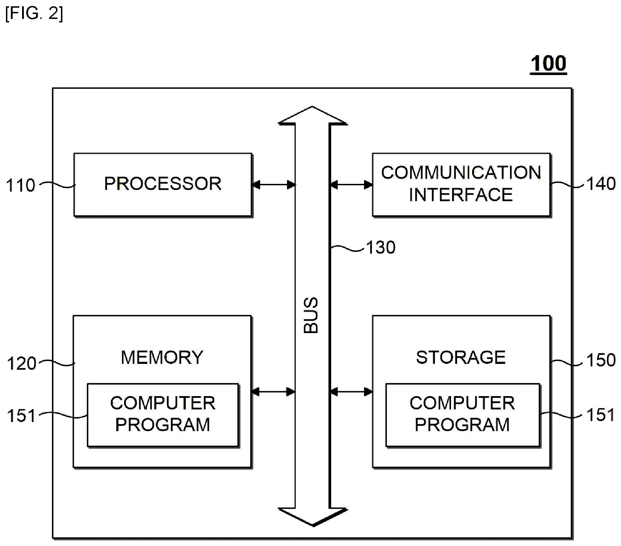

According to an embodiment, the external server 300 may be connected to the computing device 100 via the network and may store and manage various kinds of information and data required for the computing device 100 to perform a method of providing guidance on multifocal intraocular lens implantation on the basis of analysis of a patient's corneal state. Also, the external server 300 may collect, store, and manage various kinds of information and data that is derived by the computing device 100 performing the method of providing guidance on multifocal intraocular lens implantation on the basis of analysis of a patient's corneal state. For example, the external server 300 may be, but is not limited to, a storage server that is separately provided outside of the computing device 100. The method of providing guidance on multifocal intraocular lens implantation on the basis of analysis of a patient's corneal state will be described below with reference to FIG. 2.

FIG. 2 is a hardware configuration diagram of a computing device for performing a method of providing guidance on multifocal intraocular lens implantation on the basis of analysis of a patient's corneal state according to another embodiment of the present invention.

Referring to FIG. 2, according to various embodiments, the computing device 100 may include at least one processor 110, a memory 120 to which a computer program 151 into which a computer program 151 executed by the processor 110 is loaded, a bus 130, a communication interface 140, and a storage 150 in which the computer program 151 is stored. In FIG. 2, only components related to embodiments of the present invention are shown. Therefore, those skilled in the technical field to which the present invention pertains should appreciate that general-use components other than the components shown in FIG. 2 may be additionally included.

The processor 110 controls overall operation of the computing device 100. The processor 110 may include a central processing unit (CPU), a microprocessor unit (MPU), a microcontroller unit (MCU), a graphics processing unit (GPU), or any form of processor well known in the technical field of the present invention.

In addition, the processor 110 may perform computation for at least one application or program for executing a method according to embodiments of the present invention, and the computing device 100 may include at least one processor.

According to various embodiments, the processor 110 may further include a random access memory (RAM) and a read-only memory (ROM) that temporarily and/or permanently store signals (or data) processed in the processor 110. Also, the processor 110 may be implemented in the form of a system on chip (SoC) including at least one of a GPU, a RAM, and a ROM.

The memory 120 stores various kinds of data, commands, and/or information. The computer program 151 may be loaded from the storage 150 into the memory 120 to perform methods/operations according to various embodiments of the present invention. When the computer program 151 is loaded into the memory 120, the processor 110 may perform the methods/operations by executing one or more instructions constituting the computer program 151. The memory 120 may be implemented as a volatile memory such as a RAM, but the technical scope of the present disclosure is not limited thereto.

The bus 130 provides a communication function between the components of the computing device 100. The bus 130 may be implemented in various forms of buses such as an address bus, a data bus, a control bus, and the like.

The communication interface 140 supports wired or wireless Internet communication of the computing device 100. Also, the communication interface 140 may support various communication methods in addition to Internet communication. To this end, the communication interface 140 may include a communication module well known in the technical field of the present invention. In some embodiments, the communication interface 140 may be omitted.

The storage 150 may non-temporarily store the computer program 151. When the computing device 100 performs a process of providing guidance on multifocal intraocular lens implantation on the basis of analysis of a patient's corneal state, the storage 150 may store various kinds of information required for providing the process of providing guidance on multifocal intraocular lens implantation on the basis of analysis of a patient's corneal state.

The storage 150 may include a non-volatile memory, such as a ROM, an erasable programmable ROM (EPROM), an electrically erasable programmable ROM (EEPROM), a flash memory, etc., a hard disk, a removable disk, or any form of computer-readable recording medium well known in the technical field to which the present invention pertains.

The computer program 151 may include one or more instructions that, when loaded into the memory 120, cause the processor 110 to perform methods/operations according to various embodiments of the present invention. In other words, the processor 110 may perform the methods/operations according to various embodiments of the present invention by executing the one or more instructions.

In the embodiment, the computer program 151 may include one or more instructions for performing the method of providing guidance on multifocal intraocular lens implantation on the basis of analysis of a patient's corneal state including a step of acquiring ocular data generated by scanning a patient's ocular area, a step of determining the patient's corneal state by analyzing the obtained ocular data, and a step of providing guidance on multifocal intraocular lens implantation to be performed on the patient on the basis of the determined corneal state of the patient.

Steps of methods or algorithms described in connection with embodiments of the present invention may be directly implemented using hardware, implemented using software modules executed by hardware, or implemented using a combination thereof. The software modules may reside in a RAM, a ROM, an EPROM, an EEPROM, a flash memory, a hard disk, a removable disk, a compact disc (CD)-ROM, or any form of a computer-readable recording medium well known in the technical field to which the present invention pertains.

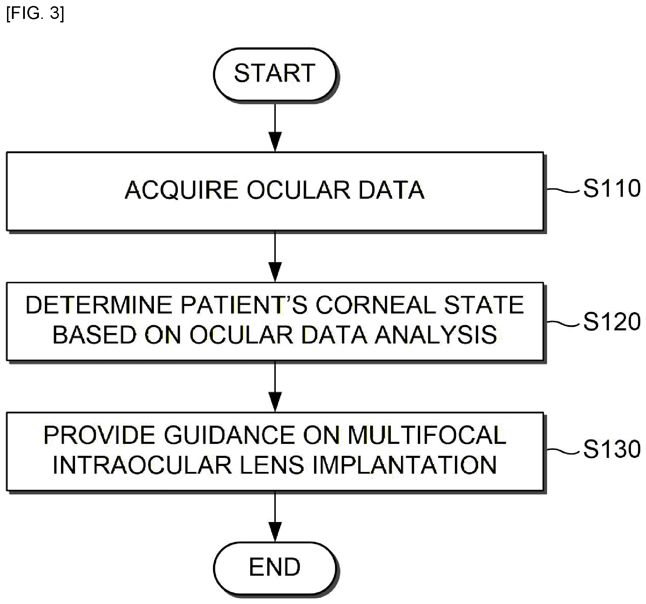

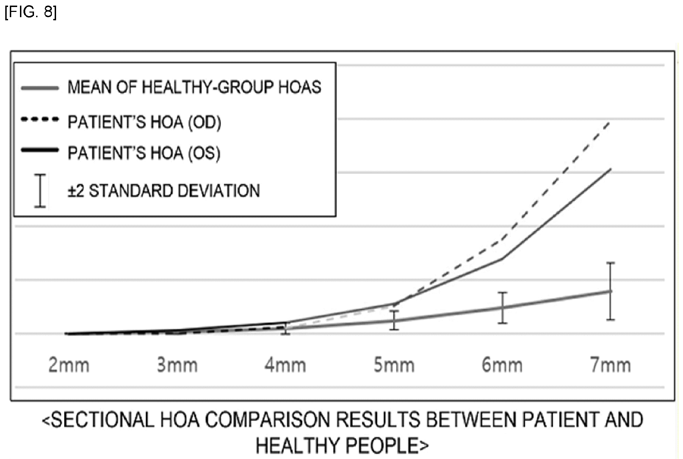

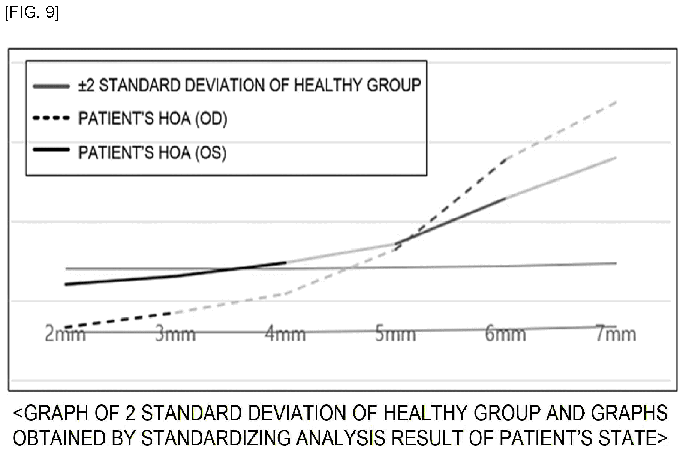

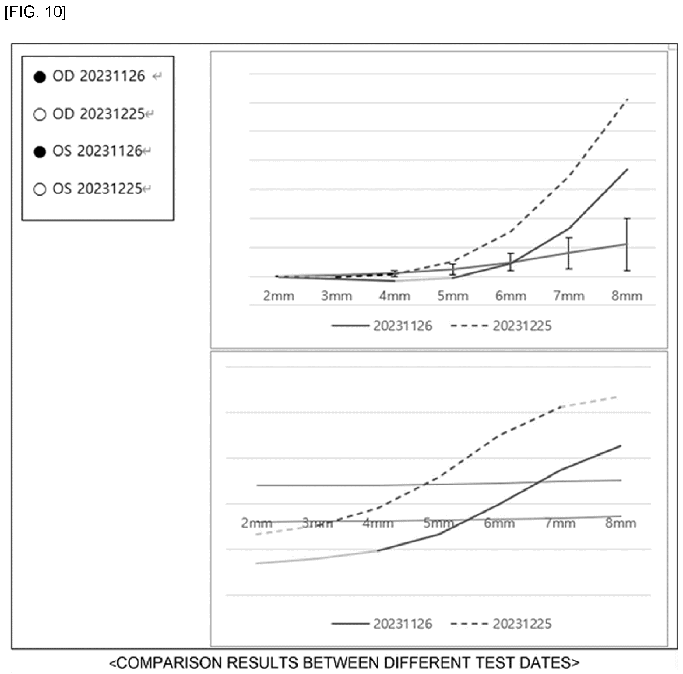

Components of the present invention may be implemented as a program (or application) and stored in a medium to be executed in combination with a computer which is hardware. Components of the present invention may be executed by software programming or software elements. Similarly, an embodiment may be implemented in a programming or scripting language, such as C, C++, Java, an assembler, etc., to include various algorithms which are embodied as combinations of data structures, processes, routines, or other programming elements. Functional aspects may be implemented using an algorithm executed by one or more processors. The method of providing guidance on multifocal intraocular lens implantation on the basis of analysis of a patient's corneal state performed by the computing device 100 will be described below with reference to FIGS. 3 to 10.

FIG. 3 is a flowchart illustrating a method of providing guidance on multifocal intraocular lens implantation on the basis of analysis of a patient's corneal state according to still another embodiment of the present invention.

Referring to FIG. 3, in step S100, ocular data generated by scanning a patient's ocular area may be acquired.

The ocular data is data acquired via a corneal topographer (e.g., Pentacam^{®}) and may be, but is not limited to, three-dimensional (3D) image data of a cornea generated using the Scheimpflug method.

The Scheimpflug method is based on the optical theory that images with a deep depth of field can be captured when a camera's lens plane, subject plane, and image plane are positioned to intersect at a specific line. Unlike general photography techniques, this method enables precise capture of depth information from a 3D subject rather than a flat subject.

In 3D corneal imaging, a Scheimpflug camera may capture high-resolution cross-sectional images of the cornea and the anterior segment of the eye, and these cross-sectional images may be combined using software to generate a 3D model of the cornea. This model accurately represents various optical and structural characteristics of the cornea including its overall shape, asymmetry, irregularity, curvature, thickness, and the like.

In step S120, the computing device 100 may determine the patient's corneal state by analyzing the ocular data acquired through step S110.

According to various embodiments, the computing device 100 may calculate a metric by analyzing the ocular data and determine the patient's corneal state using the calculated metric.

The metric is related to irregularity of the corneal surface and may be, but is not limited to, high-order aberrations (HOAs) (e.g., spherical aberrations, coma aberrations, trefoil aberrations, etc.).

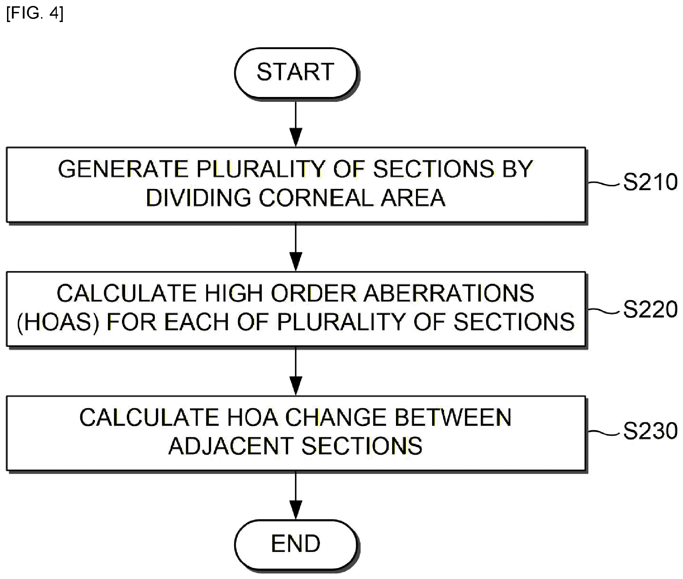

According to various embodiments, the computing device 100 may generate a plurality of sections by dividing the patient's ocular area and calculate the metric related to the irregularity of the corneal surface by analyzing ocular data corresponding to each of the plurality of sections. This will be described in further detail with reference to FIG. 4.

FIG. 4 is a flowchart illustrating a method of calculating a metric for determining a patient's corneal state according to various embodiments.

Referring to FIG. 4, in step S210, the computing device 100 may select any one of a pupil center and a corneal apex as a reference and divide the patient's corneal area at predetermined intervals, thereby generating a plurality of sections. For example, when the patient's corneal area has a radius of 7 mm, the computing device 100 may divide the area with a radius of 7 mm from the pupil center or corneal apex at intervals of 1 mm to generate a first section (a section within a radius of 2 mm), a second section (a section within a radius of 3 mm), a third section (a section within a radius of 4 mm), a fourth section (a section within a radius of 5 mm), a fifth section (a section within a radius of 6 mm), and a sixth section (a section within a radius of 7 mm), but the way of dividing the corneal area is not limited thereto.

In step S220, the computing device 100 may separately calculate HOAs for the plurality of sections as a metric related to the irregularity of the corneal surface.

As an example, the computing device 100 may calculate the HOAs for each of the plurality of sections by analyzing ocular data corresponding to each of the plurality of sections on the basis of an analysis method employing a Zernike polynomial. An HOA calculated according to the analysis method employing a Zernike polynomial may quantitatively express an HOA including a corneal refractive error by decomposing the HOA into a Zernike polynomial.

In step S230, the computing device 100 may calculate an HOA change between adjacent sections as a metric related to the irregularity of the corneal surface. For example, the computing device 100 may calculate a first HOA change on the basis of a HOA difference between the first section and the second section and calculate a second HOA change on the basis of a HOA difference between the second section and the third section, but the way of calculating HOA changes is not limited thereto.

Referring back to FIG. 3, according to various embodiments, the computing device 100 may determine the patient's corneal state using the metric calculated from the patient's ocular data.

According to various embodiments, the computing device 100 may determine the patient's corneal state depending on whether the metric calculated from the patient's ocular data are within a normal range.

More specifically, first, the computing device 100 may set a normal range using a plurality of pieces of ocular data generated by scanning eyes of healthy people with no history of ophthalmic surgery. As an example, the computing device 100 may calculate a plurality of reference metrics using a plurality of pieces of ocular data generated by scanning eyes of healthy people with no history of ophthalmic surgery and set a normal range using the plurality of reference metrics.

For example, the computing device 100 may calculate a mean of a plurality of reference metrics (e.g., HOAs and/or HOA changes) calculated from a plurality of pieces of ocular data of healthy people and set a range of ±2 standard deviations from the calculated mean of the reference metrics as a normal range.

Subsequently, the computing device 100 may determine the patient's corneal state depending on whether the metric calculated from the patient's ocular data is within the normal range.

For example, when the metric is within the normal range, the computing device 100 may determine the patient's corneal state as a normal state.

Meanwhile, when the metric deviates from the normal range, the computing device 100 may determine the patient's corneal state as an abnormal state.

According to various embodiment, when the metric deviates from the normal range, the computing device 100 may determine the patient's corneal state as an abnormal state. Here, the computing device 100 may determine the patient's corneal state as a caution state when the calculated metric is a threshold or less, and may determine the patient's corneal state as a danger state when the calculated metric exceeds the threshold.

For example, when the metric (e.g., an HOA and/or HOA change) calculated from the patient's ocular data is within a range of -3 standard deviation to -2 standard deviation from the mean of the reference metrics or exceeds +2 standard deviation and is less than or equal to +3 standard deviation, the computing device 100 may determine the patient's corneal state as a caution state.

Meanwhile, when the metric (e.g., an HOA and/or HOA change) calculated from the patient's ocular data is within a range of -3 standard deviation or less from the mean of the reference metrics or a range of greater than +3 standard deviation, the computing device 100 may determine the patient's corneal state as a danger state.

According to various embodiments, the computing device 100 may separately determine states of each of the plurality of sections generated by dividing the patient's corneal area. For example, the computing device 100 may separately set normal ranges for each of the plurality of sections using reference metrics of each of the plurality of sections calculated by analyzing ocular data of healthy people and may separately determine corneal states of each of the plurality of sections by comparing metrics of each of the plurality of sections calculated by analyzing the patient's ocular data with the normal ranges separately set for each of the plurality of sections.

According to various embodiments, the computing device 100 may determine the patient's corneal state by comparing the metric extracted from the patient's ocular data with a healthy-group normal distribution.

More specifically, first, the computing device 100 may calculate a plurality of reference metrics using a plurality of pieces of ocular data generated by scanning eyes of healthy people with no history of ophthalmic surgery and generate a healthy-group normal distribution using the plurality of reference metrics.

Subsequently, the computing device 100 may standardize the metric calculated from the patient's ocular data. Here, standardizing the metric may be calculating a Z-score of the metric but is not limited thereto.

Subsequently, the computing device 100 may determine the patient's corneal state on the basis of a location of the standardized metric in the generated healthy-group normal distribution. For example, when the standardized metric is positioned within a range of -2 healthy-group normal distribution to +2 healthy-group normal distribution, the computing device 100 may determine the patient's corneal state as a normal state. On the other hand, when the standardized metric is positioned within a range of -2 healthy-group normal distribution or less or a range of greater than +2 healthy-group normal distribution, the computing device 100 may determine the patient's corneal state as an abnormal state.

In step S130, the computing device 100 may provide guidance on multifocal intraocular lens implantation to be performed on the patient on the basis of the patient's corneal state determined through step S120.

As an example, the computing device 100 may determine prognosis of multifocal intraocular lens implantation performed on the patient on the basis of the patient's corneal state and provide the determined prognosis. For example, when an HOA of the patient's cornea deviates from ±2 standard deviation of an HOA mean of healthy people's corneas, the computing device 100 may provide a notification that there is a possibility of glare due to the multifocal intraocular lens implantation. Also, when an HOA change of the patient's cornea deviates from ±2 standard deviation of an HOA change mean of healthy people's corneas, the computing device 100 may provide a notification that there is a possibility of vision deterioration due to the multifocal intraocular lens implantation.

As another example, the computing device 100 may recommend a suitable intraocular lens for the patient on the basis of the result of determining the patient's corneal state or provide guidance on an intraocular lens that requires caution for use. For example, when the patient's corneal state is determined as a caution state, the computing device 100 may provide guidance advising against the use of diffractive intraocular lenses. Also, when the patient's corneal state is determined as a warning state, the computing device 100 may provide guidance advising against the use of any multifocal intraocular lens.

As still another example, when the patient's corneal state is determined as an abnormal state, the computing device 100 may provide guidance recommending the patient refractive surgery (e.g., topography guided surgery or wavefront guided surgery) before multifocal intraocular lens implantation.

As yet another example, the computing device 100 may visualize comparison results between the patient's corneal state and healthy people's corneal states and provide the visualized comparison results. For example, the computing device 100 may provide a user interface (UI) for visualizing comparison results between the patient's corneal state and corneal states of healthy people with no ophthalmic surgery and providing the visualized comparison results.

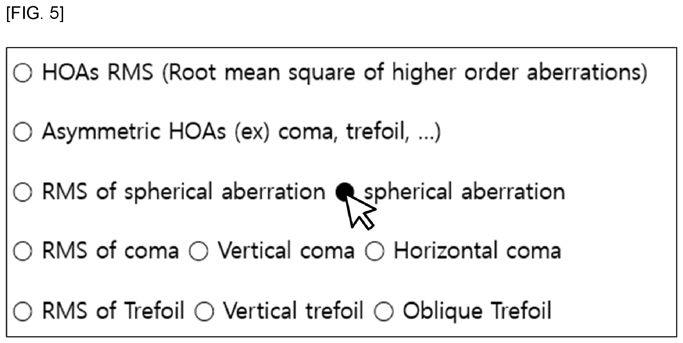

Referring to FIG. 5, the UI provided by a computing device may list and provide various kinds of metrics calculable from ocular data, and results of comparing the patient's corneal state with corneal states of healthy people on the basis of at least one metric selected by a user from a plurality of kinds of metrics may be visualized and displayed via the UI.

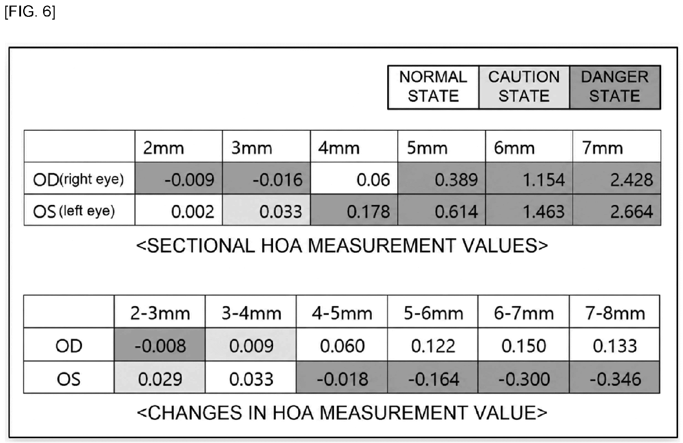

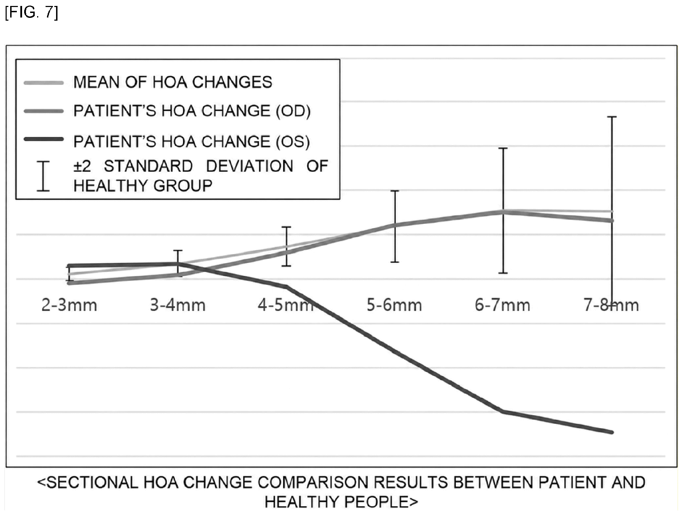

Visualizing the results of comparing the patient's corneal state with healthy people's corneal states may be, but is not limited to, displaying the results in the form of a table as shown in FIG. 6 and in the form of a graph as shown in FIGS. 7 to 10.

In various embodiments, when the patient's corneal state is separately determined for each of the plurality of sections generated by dividing the corneal area, the computing device 100 may separately provide guidance for each of the plurality of sections on the basis of the corneal states of each of the plurality of sections. Also, the computing device 100 may separately determine corneal states of the patient's left and right eyes using ocular data of each of the left and right eyes and separately provide guidance for the left and right eyes.

For example, when corneal state comparison results between the patient and healthy people are as shown in FIG. 5, the computing device 100 may provide separate guidance for the left and right eyes, such as "right eye requires topography guided surgery, Be careful using any multifocal intraocular lens" and "the left eye requires topography guided surgery, a refractive intraocular lens is usable when the photopic pupil size is 3 mm or less, Be careful using any multifocal intraocular lens when the photopic pupil size is 3 mm or more," and may provide separate guidance for each section of the left and right eyes.

The method of providing guidance on multifocal intraocular lens implantation on the basis of analysis of a patient's corneal state has been described above with reference to flowcharts shown in the drawings. Although the method of providing guidance on multifocal intraocular lens implantation on the basis of analysis of a patient's corneal state has been described as a series of blocks for clarity of description, the present invention is not limited to the order of blocks, and some blocks may be performed simultaneously or in a different order from that illustrated and described in this specification. In addition, new blocks not described in this specification and drawings may be added, or some blocks may be excluded or changed.

Although embodiments of the present invention have been described with reference to the accompanying drawings, those of ordinary skill in the technical field to which the present invention pertains should appreciate that the present invention can be implemented in other specific forms without changing the technical idea or essential characteristics thereof. Therefore, it is to be understood that the embodiments set forth herein are exemplary in all aspects and are not limiting.

## Claims

1. A method of providing guidance on multifocal intraocular lens implantation on the basis of analysis of a patient's corneal state, the method performed by a computing device and comprising:
acquiring ocular data generated by scanning a patient's ocular area;
determining the patient's corneal state by analyzing the obtained ocular data; and
providing guidance on multifocal intraocular lens implantation to be performed on the patient on the basis of the determined corneal state of the patient.

2. The method of claim 1, wherein the determining of the patient's corneal state comprises:
calculating a metric related to irregularity of a corneal surface by analyzing the acquired ocular data; and
determining the patient's corneal state using the calculated metric.

3. The method of claim 2, wherein the calculating of the metric comprises:
generating a plurality of sections by dividing the patient's corneal area at predetermined intervals on the basis of a corneal apex or a pupil center; and
calculating a plurality of high-order aberrations (HOAs) for each of the plurality of generated sections as the metric related to the irregularity of the corneal surface.

4. The method of claim 2, wherein the calculating of the metric comprises:
generating a plurality of sections by dividing the patient's corneal area at predetermined intervals on the basis of a corneal apex or a pupil center;
calculating a plurality of HOAs for each of the plurality of generated sections; and
calculating an HOA change between adjacent sections as the metric related to the irregularity of the corneal surface.

5. The method of claim 2, wherein the determining the patient's corneal state using the calculated metric comprises:
calculating a plurality of reference metrics using a plurality of pieces of ocular data generated by scanning eyes of healthy people with no history of ophthalmic surgery, and setting a normal range using the plurality of calculated reference metrics; and
determining the patient's corneal state depending on whether the calculated metric is within the set normal range.

6. The method of claim 3, wherein the determining of the patient's corneal state depending on whether the calculated metric is within the set normal range comprises:
determining the patient's corneal state as a normal state when the calculated metric is within the set normal range; and
determining the patient's corneal state as an abnormal state when the calculated metric deviates from the set normal range wherein the patient's corneal state is determined as a caution state when the calculated metric is a threshold or less, and the patient's corneal state is determined as a danger state when the calculated metric exceeds the threshold.

7. The method of claim 2, wherein the determining the patient's corneal state using the calculated metric comprises:
calculating a plurality of reference metrics using a plurality of pieces of ocular data generated by scanning eyes of healthy people with no history of ophthalmic surgery, and generating a healthy-group normal distribution using the plurality of calculated reference metrics; and
standardizing the calculated metric and determining the patient's corneal state on the basis of a location of the standardized metric in the generated healthy-group normal distribution.

8. The method of claim 1, wherein the providing of the guidance comprises determining prognosis of the multifocal intraocular lens implantation on the basis of the determined corneal state and providing guidance for information on the determined prognosis.

9. The method of claim 1, wherein the providing of the guidance comprises, when the patient's corneal state is determined as an abnormal state on the basis of the determined corneal state, providing guidance recommending the patient refractive surgery before the multifocal intraocular lens implantation.

10. The method of claim 1, wherein the providing of the guidance comprises providing guidance on use of an intraocular lens for the multifocal intraocular lens implantation on the basis of the determined corneal state wherein guidance on cautious use of a diffractive intraocular lens is provided when the determined corneal state is a caution state, and guidance on cautious use of any intraocular lens is provided when the determined corneal state is a warning state.

11. The method of claim 1, wherein the providing of the guidance comprises providing a user interface (UI) for visualizing comparison results between the determined corneal state and corneal states of healthy people with no history of ophthalmic surgery and outputting the visualized comparison results.

12. A computing device for performing a method of providing guidance on multifocal intraocular lens implantation on the basis of analysis of a patient's corneal state, comprising:
a processor;
a network interface;
a memory; and
a computer program loaded into the memory and executed by the processor,
wherein the computer program comprises:
an instruction to acquire ocular data generated by scanning a patient's ocular area;
an instruction to determine the patient's corneal state by analyzing the obtained ocular data; and
an instruction to provide guidance on multifocal intraocular lens implantation to be performed on the patient on the basis of the determined corneal state of the patient.

13. A computer program stored on a computer-readable recording medium to perform, in combination with a computing device, a method of providing guidance on multifocal intraocular lens implantation on the basis of analysis of a patient's corneal state, wherein the method comprises:
acquiring ocular data generated by scanning a patient's ocular area;
determining the patient's corneal state by analyzing the obtained ocular data; and
providing guidance on multifocal intraocular lens implantation to be performed on the patient on the basis of the determined corneal state of the patient.
